(19) Europäisches Patentamt
European Patent Office
Office européen des brevets

(11) **EP 3 426 827 B1**

(12) **EUROPEAN PATENT SPECIFICATION**

(45) Date of publication and mention
of the grant of the patent:
**28.10.2020 Bulletin 2020/44**

(21) Application number: **17782056.0**

(22) Date of filing: **10.04.2017**

(51) Int Cl.:
*A61L 15/26* (2006.01)      *A61L 27/18* (2006.01)
*A61L 27/36* (2006.01)      *D04H 1/4266* (2012.01)
*D04H 1/435* (2012.01)      *D04H 1/728* (2012.01)
*D01D 5/34* (2006.01)      *D01F 8/02* (2006.01)
*D01F 8/04* (2006.01)      *A61L 15/40* (2006.01)

(86) International application number:
**PCT/IL2017/050442**

(87) International publication number:
**WO 2017/179055 (19.10.2017 Gazette 2017/42)**

(54) **JELLYFISH EXTRACT NANOFIBERS**

NANOFASERN AUS QUALLENEXTRAKT

NANOFIBRES À BASE D'EXTRAIT DE MÉDUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.04.2016 US 201662320547 P**

(43) Date of publication of application:
**16.01.2019 Bulletin 2019/03**

(73) Proprietor: **Ramot at Tel-Aviv University Ltd.**
**6139201 Tel-Aviv (IL)**

(72) Inventors:
• **RICHTER, Shachar**
**7680400 Mazkeret Batia (IL)**
• **NUDELMAN, Roman**
**6139201 Tel Aviv (IL)**
• **GULAKHMEDOVA, Tamilla**
**6139201 Tel Aviv (IL)**

(74) Representative: **CMS Cameron McKenna Nabarro Olswang LLP**
**Cannon Place**
**78 Cannon Street**
**London EC4N 6AF (GB)**

(56) References cited:
WO-A1-2007/029913      WO-A1-2014/005090
WO-A1-2014/106830      WO-A1-2016/025945

• CHOI D.J. ET AL.: 'Bioactive fish collagen/polycaprolactone composite nanofibrous scaffolds I fabricated by electrospinning for 3D cell culture' JOURNAL OF BIOTECHNOLOGY, [Online] vol. 205, 21 January 2015, ISSN 0168-1656 pages 47 - 58, XP029240259 ISSN: 0168-1656 Retrieved from the Internet: <URL:http://dx.doi.org/10.1016/jjbiotec. 2015.01.01 7>
• GUMUSDERELIOGLU M. ET AL.: 'A novel dermal substitute based on biofunctionalized I electrospun PCL nanofibrous matrix' JOURNAL OF BIOMEDICAL MATERIALS RESEARCH vol. 98 A, no. 3, 09 June 2011, ISSN 1552-4965 pages 461 - 472, XP055433853
• ZHANG Y.Z. ET AL.: 'Characterization of the Surface Biocompatibility of the Electrospun PCL-Collagen Nanofibers Using Fibroblasts' BIOMACROMOLECULES vol. 6, no. 5, 27 July 2005, ISSN 1526-4602 pages 2583 - 2589, XP055038547
• JANAIRO R.R.R . ET AL.: 'Mucin covalently bonded to microfibers improves the patency of vascular grafts' TISSUE ENGINEERING vol. 20, no. ISSUES, 01 January 2014, ISSN 2152-4955 pages 285 - 293, XP055433866

**EP 3 426 827 B1**

**Description**

**RELATED APPLICATIONS**

The present application claims benefit under 35 U.S. C. 119(e) from U.S. Provisional Application 62/320,547 filed April 10, 2016.

**BACKGROUND**

Jellyfish are members of the phylum Cnidaria of aquatic organisms, which typically live in salt water seas and oceans. Jellyfish have tentacles, which may contain stinging structures, comprising venom, and a gelatinous bell. Jellyfish tend to drift, while feeding on plankton, fish and sometimes other jellyfish. In certain locations, jellyfish tend to drift in groups consisting of large numbers of jellyfish, called blooms.

Large jellyfish blooms may be detrimental to humans. When blooms approach coastal bathing areas, jellyfish may release their venom into sea water or sting humans upon contact, often causing an unpleasant allergic reaction, which may be severe or even deadly in the case of certain jellyfish. In addition, blooms impact fishing industries, by eating commercial fish and by becoming entangled in fishing nets. Another negative impact of jellyfish blooms is clogging of industrial equipment. Jellyfish have been implicated in damage to power plants, desalination plants and ship engines that rely on sea-water intake.

Industries which rely on salt-water intake, such as power plants, may need to remove large quantities (multiple tons per day) of jellyfish from water intake systems, to ensure proper functioning of these systems. Once the jellyfish are removed, they cause an ecological problem, as they need to be disposed of and regulations often prohibit dumping them back into the sea. As a result, they require shipment to landfills for burial. Disposal is often difficult, because once jellyfish are removed from water, they begin to decay and emit an unpleasant smell. JANAIRO R.R.R . ET AL.: "Mucin covalently bonded to microfibers improves the patency of vascular grafts",TISSUE ENGINEERING, vol. 20, no. ISSUES, 1 January 2014 (2014-01-01), pages 285-293; discloses electrospun PCL nanofibres functionalized with mucin and used as scaffold.

However, compositions from jellyfish such as jellyfish collagen may be useful. The jellyfish compositions carry less risk of prion and viral contamination than compositions originating from other sources.

**SUMMARY**

An aspect of an embodiment of the disclosure relates to nanofibers comprising a jellyfish biomass-derived composition (which may be referred to herein as a jellyfish extract). as defined in claim 1.

For convenience of presentation, the nanofibers in accordance with an embodiment of the disclosure may be referred to herein as "jellyfish nanofibers".

The jellyfish nanofibers may have a diameter of between 40 nanometers (nm) and 900 nm, optionally between 150 and 250 nm. The length of the jellyfish nanofibers may be greater than 1 millimeter (mm), greater than 1 centimeter (cm) or greater than 1 meter (m). The jellyfish nanofiber may be combined, or overlaid in a random or semi-random matrix structure to form a nanofiber scaffold.

The jellyfish extracts comprise at least one glycoprotein extracted from jellyfish tissue. The at least one glycoprotein comprises Q-mucin, also known as qniumucin.

The jellyfish nanofibers further comprise a jellyfish-derived collagen and at least one non-jellyfish-derived electrospinnable polymer, which may be referred to herein as a "support polymer". Optionally, the support polymer comprises polycaprolactone (PCL) and/or PCL-calcium phosphate.

Jellyfish nanofibers according to embodiments of the disclosure are formed through electrospinning of a mixture of a jellyfish-derived composition with a support polymer. The electrospinning may be performed using a voltage of between 10 and 19 kilovolts (kV).

According to an embodiment of the disclosure, a non-woven material may be formed from a plurality of jellyfish nanofibers, for example, to form a matrix of stacked nanofibers, which may be used as a biological matrix for treating a disease or a wound. The biological matrix may be biodegradable and may decompose in a human body. Optionally, the plurality of nanofibers is combined with, or formed together with, an additive such as an antimicrobial agent, thereby forming nanofibers suitable for wound dressing for treating wounds. In addition, nanofibers according to embodiments of the invention may be used for medical implants, prosthetics, filtration units, tissue engineering, cosmetics and nanosensors.

Further embodiments of the invention relate to methods for manufacture of nanofibers comprising jellyfish extract, the methods comprising electro spinning.

In the discussion unless otherwise stated, adjectives such as "substantially" and "about" modifying a condition or relationship characteristic of a feature or features of an embodiment of the invention, are understood to mean that the condition or characteristic is defined to within tolerances that are acceptable for operation of the embodiment for an application for which it is intended. Unless otherwise indicated, the word "or" in the specification and claims is considered to be the inclusive "or" rather than the exclusive or, and indicates at least one of, or any combination of items it conjoins.

This summary is provided to introduce a selection of concepts in a simplified form that are further described below in the detailed description. This summary is not intended to identify key features or essential features of the claimed subject matter, nor is it intended to be used to limit the scope of the claimed subject matter.

**BRIEF DESCRIPTION OF FIGURES**

Non-limiting examples of embodiments of the invention are described below with reference to figures attached hereto that are listed following this paragraph. Identical structures, elements or parts that appear in more than one figure are generally labeled with a same numeral in all the figures in which they appear. Dimensions of components and features shown in the figures are chosen for convenience and clarity of presentation and are not necessarily shown to scale.

Fig. 1A depicts an electron scanning micrograph of a scaffold formed using an electrospinning apparatus, by electrospinning a solution comprising PCL without jellyfish extract;

Fig. 1B depicts an electron scanning micrograph of a nanofiber scaffold mat formed using an electrospinning apparatus, by electrospinning a solution comprising PCL and a jellyfish extract, according to embodiments of the invention;

Figs. 2A-2D depict histograms showing fiber diameter distribution for jellyfish extract-containing nanofibers prepared using electrospinning methods at needle to collector distances of 12 cm (Fig. 2A), 13 cm (Fig. 2B), 14 cm (Fig. 2C) and 16 cm (Fig. 2D);

Fig. 3 depicts a graph showing release of tetracycline over time into solution from a nanofiber scaffold mat comprising PCL-jellyfish extract nanofibers doped with tetracycline;

Figure 4A is a graph plotting evaluated wound closure (%) over time (days) for a mouse whose wounds are treated with an applied jelly fish extract-PCL nanofiber antimicrobial wound dressing;

Figure 4B is a graph plotting evaluated wound closure (%) over time (days) for another mouse whose wounds are treated with an applied jelly fish extract-PCL nanofiber antimicrobial wound dressing;

Figure 4C is a graph plotting evaluated wound closure (%) over time (days) for yet another mouse whose wounds are treated with an applied jelly fish extract-PCL nanofiber antimicrobial wound dressing;

Figure 4D is a graph plotting evaluated wound closure (%) over time (days) for yet another mouse whose wounds are treated with an applied jelly fish extract-PCL nanofiber antimicrobial wound dressing;

Figure 4E is a graph plotting evaluated wound closure (%) over time (days) for yet another mouse whose wounds are treated with an applied jelly fish extract-PCL nanofiber antimicrobial wound dressing;

Figure 5A presents a structure of jellyfish extract-PCL nanofiber during and after the electrospinning process in accordance with an embodiment of the disclosure;

Figure 5B is an ESEM image of damaged jellyfish extract-PCL nanofibers exhibiting exposed inner core PCL material (scale bar 5 $\mu$m);

Figure 5C depicts fiber size distribution of jellyfish extract-PCL nanofibers before and after enzymatic degradation by pepsin;

Figure 5D is a confocal fluorescence micrograph of DTAF labeled jellyfish extract-PCL nanofibers and Figure 5E is a confocal fluorescence micrograph of reference non-labeled fibers;

Figure 6A is an electron microscope micrograph of electrospun nanofibers made from Korean jellyfish extract;

Figure 6B is an electron microscope micrograph of silver nanoparticles' nucleation on nanofibers made from Korean jellyfish extract;

Fig. 7A schematically shows an exploded view of a four-layer composite wound dressing in accordance with an embodiment of the disclosure; and

Fig. 7B schematically shows a completed four-layer composite dressing placed over a wound on a skin of a subject.

**DETAILED DESCRIPTION**

In the detailed description below, improved nanofibers comprising jellyfish extract, also referred to below as "jellyfish nanofibers", and processes for their synthesis are described. In addition, biological matrices such as wound dressings comprising jellyfish nanofibers are described.

Jellyfish nanofibers according to embodiments of the disclosure provide a number of advantages including, but not limited to: a. they are made from an inexpensive, readily available source, b. harvesting jellyfish for production of jellyfish protein polymer provides an environmentally friendly method for removal and disposal of potentially harmful jellyfish, c. products made from the nanofibers can provide benefits in treatment of various indications, including wound healing and d. products made from the jellyfish nanofibers may be impregnated with additives such as an antimicrobial agent, and the additive may be released from the product over time as the product is in contact with a biological tissue.

In an embodiment of the disclosure, the jellyfish extract is an alcohol extract of jellyfish biomass. Optionally the alcohol is ethanol. Optionally, the jellyfish extract is extracted by incubating the jellyfish biomass in between 70% and 100% ethanol, optionally about 96% ethanol. Optionally, the jellyfish biomass comprises or consists of a bell portion of a jellyfish. Optionally, the jellyfish is *Nemopilema nomurai* or *Rhophalema Nomadica.*

The jellyfish extract comprises Q-mucin. In an embodiment of the disclosure, between 1% and 15% of the dry weight of the jellyfish extract consists of Q-mucin. Optionally, between 1% and 3% of the dry weight of the jellyfish extract consists of Q-mucin.

"Electrospinning" is a process derived from the term, "electrostatic spinning" which refers to a process in which a high voltage is applied between an outlet for ejection of a polymeric solution and a collector. Typically, an electrospinning setup comprises a high voltage power supply, the polymeric solution in a syringe tube with a blunt metal needle, and a collector plate. One electrode may be connected to the needle and another electrode may be connected to the collector plate.

Under specific conditions, a fiber may be formed using this process. The fiber may be between 10 nanometers (nm) and 1000 nm in diameter. Nanofibers in this range may have significant technological impact due to their characteristics, such as relatively high surface to volume ratio, flexibility, mechanical performance and other physical and chemical characteristics appropriate for various technological and industrial applications. In an embodiment of the disclosure, the jellyfish nanofiber is between 150 nm and 900 nm in diameter.

The electrospinning process typically does not require the use of coagulation chemistry or high temperatures to produce solid threads from solution which is an advantage for forming nanofibers comprising labile materials such as jellyfish glycoprotein.

We have now discovered by experimentation conditions under which smooth nanofibers comprising jellyfish extracts are produced, advantageously with reduced presence of beads.

Some embodiments have improved wettability.

Some embodiments further comprise antimicrobial agents embedded in the nanofibers. These embodiments may facilitate improved healing.

Optionally, the electrospinnable polymers in accordance with an embodiment of the disclosure comprises one or more of the biodegradable polymers: DegraPol® (degradable block polyesterurethane), Pellethane 2363-80A (degradable polyetherurethane), Poly(3 - hydroxybutyrate -co-3-hydroxyvalerate), Poly(3- hydroxybutyrate), Poly(D,L-lactide -co - trimethylene carbonate, Poly(D,L-lactide)-poly(ethylene glycol), Mw = 51 kDa, Poly(L-lactic acid)-co-(caprolactone), 70:30 Mw 150 kDa, Poly(L-lactic acid)-co-(glycolic acid), 70:30, Poly(propylene carbonate), Mw 699 000 g/mol, Poly(y-stearyl-L-glutamate), Poly-L-lactide 1.09 dL and Poly[(alanino ethylester)0.67 (glycino ethyl ester)0.33 phosphazene].

Jellyfish nanofibers in accordance with an embodiment of the disclosure may be collected to produce a jellyfish nanofiber scaffold mat, which may be used as a wound dressing material, a tissue engineering scaffold matrix, a filtration membranes, a catalysis matrix, a medical textile, and a drug delivery composition.

In an embodiment of the disclosure, the jellyfish nanofiber scaffold mat is loaded with an antimicrobial agent. Optionally the antimicrobial agent comprises silver nanoparticles, a tetracycline, or other antimicrobial compositions known in the art.

In an embodiment of the disclosure, the jellyfish nanofiber scaffold mat is loaded with a hydrogel composition. Optionally, the hydrogel composition comprises a sodium alginate gel.

In an embodiment of the disclosure, a plurality of jellyfish nanofiber scaffold mats may be arranged as layers comprised in a composite jellyfish wound dressing.

A composite jellyfish wound dressing in accordance with an embodiment of the disclosure comprises: a contact layer; an intermediate layer; and an external layer, wherein each of the three layers comprise at least one jellyfish nanofiber scaffold mat.

In an embodiment of the disclosure, the contact layer is configured to contact a skin of a subject, and comprises a jellyfish nanofiber scaffold mat in accordance with an embodiment of the disclosure.

In an embodiment of the disclosure, the intermediate layer optionally comprises an antimicrobial layer comprising a jellyfish nanofiber scaffold mat loaded with an antimicrobial agent. Additionally, or alternatively, the intermediate later comprises a hydrogel layer comprising a jellyfish nanofiber scaffold mat loaded with a hydrogel composition.

Optionally, the intermediate layer comprises two layers: an antimicrobial layer and a hydrogel layer, such that the composite jellyfish wound dressing comprises four layers: a contact layer, an antimicrobial layer, a hydrogel layer, and an external layer.

In an embodiment of the disclosure, the external layer

Examples described below in the following chapters include:

Examples 1A-1F: Examples of jellyfish nanofibers and production methods therefor;
Examples 2A-2H: Physical characterization of jellyfish extract/ PCL nanofibers;
Examples 3A-3D: Testing of biological properties of jellyfish nanofibers;
Example 4: Jellyfish nanofibers produced from a different jellyfish species; and
Examples 5A-5B: Testing of jellyfish nanofiber wound dressing.
Example 1A: Formation of jellyfish extracts from whole jellyfish

Raw jellyfish of *Rhophalema Nomadica* species were caught near the Israel Mediterranean sea shore. Tentacles and oral arms containing poison cells were separated from the protein-rich jellyfish bell. The bell was processed and cut with a standard food blender on maximum speed. Jellyfish bell mass was then poured into a plastic container and 96% technical grade ethanol was added at a ratio of 1:3 weight-per-weight (w/w) jellyfish bell mass to ethanol.

The jellyfish bell mass and ethanol mixture was stored at -4°C (degrees Celcius) and left for 24-72 hours (hr) for creation of bell residue. Bell residue was extracted with preparative centrifugation process (centrifuge) using the parameters: 5000 G (gravitational force) at 4°C for 30 minutes (min). Some residual ethanol remained in the resulting jellyfish extract. The extract was allowed to dry in a fume hood, then stored at -8°C until further use. The jellyfish extract prepared as described above is referred to herein below as Extract A.

Additional or alternative methods for forming jellyfish extracts:
*Rhopilema nomadica* jellyfish were collected from the shore and from the sea in Tel Aviv, Israel. The jellyfish were washed in cold water, and tentacles were removed. The jellyfish were cut into pieces and blended in a blender for approximately 3 minutes. The blend was filtered and separated into a liquid solution/suspension and gel-like material using a coarse strainer, having holes approximately 1 mm in size.

Gel-like material which remained in the strainer was washed by combining with 3 volumes of ethanol for two hours, centrifuged at 4°C for 15 minutes at 10,000 g (gravitational accelerations) to remove residual water and ethanol under vacuum, then transferred to a rotary evaporator for 8 hours, and then subjected to a higher vacuum for overnight. The remaining solid was lyophilized and then frozen. The extract is referred to as Extract B.

The liquid which was removed from the strainer was added to 3 volumes of ethanol, transferred to cold storage and subjected to centrifugation at 4°C for 15 minutes at 10,000 g. The solids were then lyophilized and frozen. The extract is referred to as Extract C.

In an alternative preparation the jellyfish were washed in cold water, and tentacles were removed. The jellyfish were cut into pieces and blended in a blender for approximately 3 minutes. The mixture was refrigerated overnight with about 80% by volume ethanol. The mixture was then dried in an evaporator, with some liquid remaining. The extract is referred to as Extract D.

Example 1B: Preparation of jellyfish Q-mucin

The preparation comprises: Swelling the jellyfish with water; cutting the swollen jellyfish; subjecting the swollen pieces to extraction with a salt water having a small concentration by shaking the pieces in the salt water; adding to the extract ethanol in an amount of 1-5 times the amount of the extract to produce precipitate in the form of a gel; centrifugal separation of the gel precipitate; dissolving the precipitate in water to form a supernatant and separating the supernatant; purifying the supernatant by dialysis; and freezing and drying the purified supernatant.

Example 1C: Preparation of PCL polymer solution and mixing with jellyfish extract

Polycaprolactone (PCL) polymer solutions were prepared in either 25% w/v or 16% w/v concentration by dissolving 0.5 grams (g) of PCL polymer pellets in either 2 or 3 milliliter (ml) of glacial acetic acid (98%) while stirring vigorously and heating at 70°-80° C until full dissolution of the polymer. Jellyfish extract solution was prepared by dissolving of either 3 or 4 g of a dried extract A derived from *Rhophalema Nomadica,* as described above in Example 1A, in either 2 or 3 ml of glacial acetic acid. The Jellyfish extract-acetic acid solution was added to the PCL solution and the resulting mixture, about 6 ml in volume, was stirred until complete homogenization. The homogenized mixture prepared as described above may be referred to hereinafter as the Polymer/Jellyfish extract mix, or "PJ mix". The ratio of jellyfish extract to PCL as in the PJ mix prepared as described above is between 6:1 and 8:1.

In an exemplary PJ mix, the final PCL concentration was 8.3% w/v, and the concentration of the jellyfish extract was 66% w/v.

Alternate solvents may be used instead of acetic acid, for example trifluoroethanol, or 1,1,1,3,3,3,-hexafluoro-2-propanol,. Optionally, formic acid and/or ethyl acetate is mixed with acetic acid in preparing the PJ mix. Alternate polymers which may be used include but are not limited to the electrospinnable polymers described hereinabove. Particular examples include poly vinyl alcohol, poly lactic acid, poly ethylene glycol, PLGA poly(lactic-co-glycolic acid) and PGA (poly glycolide).

Example 1D: PCL-Jellyfish extract Nanofiber production

The PJ mix produced in accordance with example 1C was inserted into an electrospinning setup comprising: A high voltage power supply (maximum output 30 kV), Bertan model 230R-30kV; a high precision syringe pump (mrc SYP-01 multi-mode laboratory syringe pump); a needle connected to the syringe (20-23 gauge), a clear polyacrylate electrospinning setup box, crafted to contain within it the syringe pump and collector surface and a hot plate (mrc model MUSH-10).

Experimental parameters may be varied during the spinning process. The variation may impact the fiber density, alignment, droplet formation, bead formation and fiber size. In an embodiment parameters for forming jellyfish extract/ PCL nanofibers included: a voltage of between 10 and 23 kV; a distance between 12-16 cm between the needle end and the collector. Flow speed of solution through the electrospinning setup nozzle was about 5-10 microliters/minute ($\mu$l/min). The ratio of jellyfish extract to PCL in the example PJ mix was between 6:1 and 8:1, by dry weight of the jellyfish extract and PLC, respectively.

In embodiments of the disclosure, the ratio of jellyfish extract to PCL in the PJ mix by dry weight may be between 1:1 and 10:1.

A Nanofiber scaffold mat was collected on the collector plate and stored for further use at room temperature.

Nanofibers formed according to various solution and electrospinning parameters were prepared according to table 1.

Table 1 shows various experimental parameters of the electrospining process that was used in order to make various jellyfish nanofibers. For the exemplary jellyfish nanofibers shown in Table 1, the voltage used was between 10kV and 20kV, the speed flow was between 2 $\mu$l/min and 8 $\mu$l/min, the distance was between 11 cm and 18 cm, the Jellyfish extract to PCL ratio was between 1:1 and 5:1.

Table 1:

| Distance (cm) | Speed flow ($\mu$l/min) | Voltage (kV) | Solvent volume (ml) | Jellyfish extract/PCL (w%/w%) |
|---|---|---|---|---|
| 12 | 8 | 20 | 5 | 7%/7% |
| 12.5 | 5 | 15 | 5 | 14%/7% |
| 12 | 5 | 20 | 5 | 10.5%/7% |
| 12 | 5 | 20 | 5 | 10.5%/7% |
| 17 | 7-9 | 16-22.5 | 5 | 35%/7% |
| 13 | 7-9 | 10.3-14 | 5 | 35%/7% |
| 12-16 | 7-9 | 16 | 4 | 35%/7% |

Example 1E: Nanofiber doping with antimicrobial materials

In order to prepare jellyfish extract/ PCL nanofibers doped with silver nanoparticles, a nanofiber scaffold mat was formed by electrospinning as described above until a scaffold of jellyfish nanofibers having a thickness of between 100-150 microns was formed. Silver ion solution was formed using $AgNO_3$ in various concentrations 0.005 molar (M), 0.001M 0.05M, 0.01M. The solution was dissolved until complete dissolution for about 10 min. The nanofiber scaffold was washed with deionized water to remove any residual salts. The nanofiber scaffold was introduced into the silver ion solution and left for 2 hr in the dark. The nanofiber scaffold was removed from the silver ion solution, washed with deionized water, and dried under nitrogen. The nanofiber scaffold was immersed in borate buffer solution 20 millimolar (mM) pH=9 for 24 hr. The silver doped nanofiber scaffold was washed with deionized water and dried in a fume hood at room temperature.

In order to prepare nanofibers doped with a tetracycline antimicrobial agent, a nanofiber scaffold mat was formed (as described above). A solution of 0.0001M tetracycline in water was formed. Nanofiber scaffold mat was immersed in the tetracycline solution for 2 hr. The tetracycline doped nanofiber scaffold was removed and dried. In accordance with an embodiment of the disclosure, other antimicrobial agents known in the art may be used in addition to or as an alternative to a tetracycline.

In an alternative method to form nanofibers doped with silver nanoparticles, formed within jellyfish extract/ PCL nanofibers, the following method was used. silver nitrate was added to the polymer solution before formation of nanofibers, at a concentration of 0.0001M.

Example 1F: Nanofiber doping with hydrogel composition

In order to prepare jellyfish extract/ PCL nanofibers loaded with hydrogel, a nanofiber scaffold mat was formed by electrospinning as described above until a scaffold of jellyfish nanofibers having a thickness of between 100-150 microns was formed. Hydrogel material was prepared by dissolving 0.2 gram of sodium alginate in 20 ml of deionized water and dissolving of 1.5 gram of calcium chloride in 50ml of deionized water. The previously prepared jellyfish extract/ PCL nanofiber scaffold mat was placed in the calcium chloride solution of 10 minutes and left to dry for 1 hour under fume hood. The dried nanofibers scaffold mat loaded with calcium chloride ions was then placed in the sodium alginate solution for 30 minutes until hydrogel layer formation. The hydrogel-loaded nanofiber scaffold mat was then placed in 8°C cooling for overnight drying.

Examples 2: Physical testing of jellyfish nanofibers

Nanofibers formed in accordance with the above examples were tested to determine various physical parameters including nanofiber diameter. The impact of voltage on nanofiber formation was tested for the voltage range of between 10 kV and 22.5 kV. It was found that using a voltage of under 10 kV led to droplet, not fiber formation. It was found that using voltage above 22.5kV led to accumulation of a relatively unstable mass of polymer at the collector.

Fiber size distribution data was obtained by counting >100 individual nanofibers from environmental scanning electron microscopy (ESEM) images of each sample in different areas for statistical values by scandium image processing program. ESEM apparatus used was: Quanta 200 FEG Environmental SEM. Operational voltage of 20kV under high vacuum, operational voltage of 10-15kV in low vacuum. Spot size 3, chamber pressure 70 pascal (pA). Spattering of 10 nm gold was required for enhanced contrast and resolution, performed at SPI sputter in 1.2 kV for 120 seconds (sec).

Fiber size distribution data of jellyfish extract/ PCL nanofibers synthesized in accordance with methods described above shows presence of nanofibers with minimum diameter of 40 nm and maximum diameter of 900 nm for various feed-flows and voltages. Most of the counted nanofibers have diameter between 150-250 nm. The relatively larger nanofibers having diameters greater than 600 nm often show presence of several nanofibers bundled or fused together.

In addition, polymer mixtures comprising PCL only were electrospun under conditions similar to those used for electrospinning polymer mixtures comprising a jellyfish extract/ PCL solution. The solution for making PCL (without jellyfish extract) nanofibers was formed by adding 0.5 g of PCL to 6 ml of acetic acid, and heating the liquid to about 70°C and stirring until the PCL dissolved. Electrospinning was performed using the electrospinning experimental setup described above, at 16 kV, with a needle to collector distance of 13 cm and a flow speed of $4\mu$l/min.

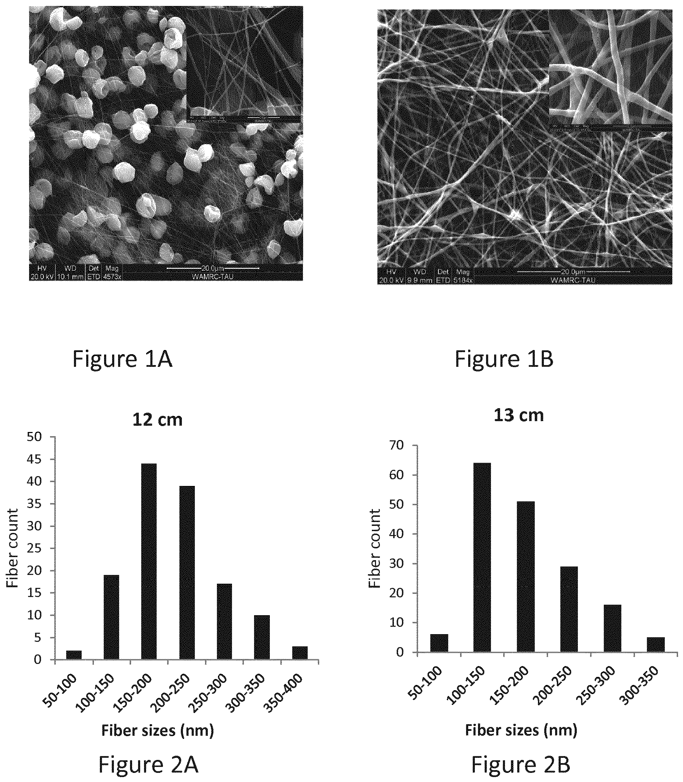

While the jellyfish extract/ PCL solution was successfully formed into smooth nanofibers with minimal amounts of beads, the PCL solution without jellyfish extract formed numerous beads with some nanofiber net formations between the beads under the same conditions. ESEM images of the bead-filled fiber net formed from PCL solution and of smooth nanofibers made from jellyfish extract/ PCL solution are presented in Figs. 1A and 1B respectively.

Example 2A: Physical characterization of jellyfish extract/ PCL nanofibers.

Nanofibers were synthesized in accordance with methods described above, using a 35%/7% jellyfish extract/ PCL solution, with a needle to collector distance of 13 cm and a flow speed of $4\mu$l/min , while varying the voltage of the electrospinning experimental setup described above:

The average diameter under various voltage conditions is shown in Table 2 below.

Table 2:

| Voltage (kV) | Average diameter (nm) | Standard deviation (nm) |
|---|---|---|
| 10 | 252.4 | 112.9 |
| 10.3 | 202.44 | 65.1 |
| 11 | 236.82 | 89.64 |
| 11.4 | 268.72 | 116.58 |
| 11.7 | 251.18 | 83.02 |
| 12.4 | 200.6 | 71.8 |
| 12.8 | 275.4 | 92.34 |
| 13.2 | 298.5 | 84.7 |

(continued)

| Voltage (kV) | Average diameter (nm) | Standard deviation (nm) |
|---|---|---|
| 13.5 | 235.6 | 74.1 |
| 14 | 262.9 | 71.3 |
| 16 | 186.63 | 52.99 |
| 17 | 258.3 | 80.9 |
| 18.5 | 197.1 | 103.5 |
| 19 | 338.7 | 135.6 |
| 20 | 355 | 135.22 |
| 21.5 | 360 | 129.34 |
| 22 | 361 | 133.547 |
| 22.5 | 422.1 | 198.1 |

As shown in Table 2, nanofibers formed with voltages under 19kV have almost a constant average size. In fact, averaging the average diameters of nanofibers formed under voltages below 19kV gave an average diameter of 242.6 nm with a standard deviation of $\pm$36nm only. When voltage is increased above 19kV a sharp increase in nanofiber size is evident.

Nanofibers were synthesized in accordance with methods described above, using a 35%/7% jellyfish extract/ PCL solution, with a needle to collector distance of 13 cm and a voltage of 16 kV. Modifying the distance between the nozzle of the needle and collector plate as shown in Table 3 had no significant effect on the nanofiber diameter within the tested range.

Table 3:

| Nozzle-collector distance (cm) | Fiber average diameter (nm) |
|---|---|
| 12 | 206 |
| 13 | 172 |
| 14 | 213.2 |
| 16 | 186.2 |

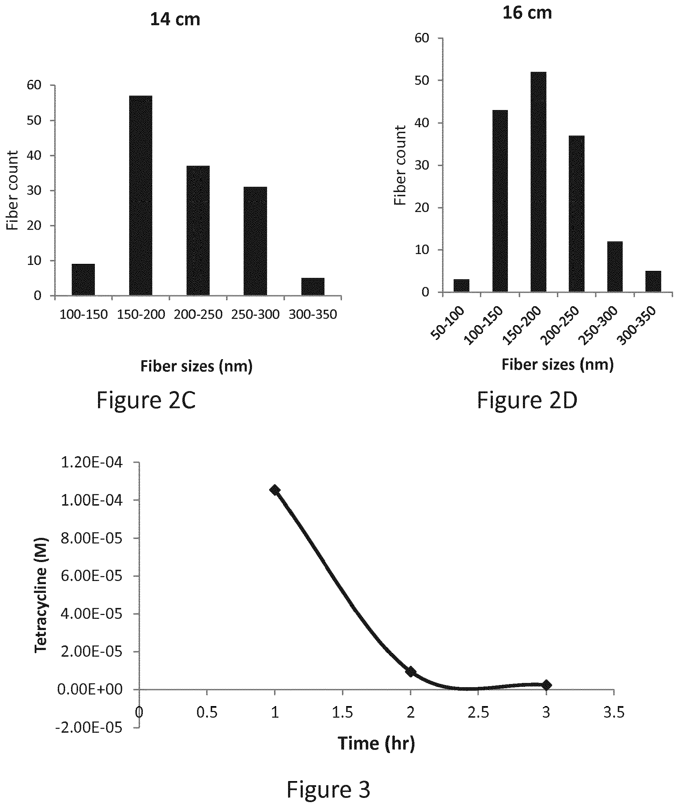

Distributions of fiber diameters are shown in Fig. 2A-2D, each figure for a different nozzle-collector distance.

Example 2B: Infrared (IR) evaluation

Attenuated total reflection (ATR) Infrared (IR) spectroscopy analyses were carried out at room temperature on a Tensor 27, Platinum ATR apparatus. All the spectra were taken via attenuated total reflection method with a resolution of 1 cm$^{-1}$ and 16 scans.

IR spectrum of PCL blend pellets (in acetic acid solvent) gave distinct footprints of the material at the following wavelengths: 2943 cm$^{(-1)}$, 2864 cm$^{(-1)}$ which are attributed to $CH_2$ vibrations. Further peaks were identified at 1720 cm$^{(-1)}$ due to C=O vibration, 1239 cm$^{(-1)}$ due to O=C---O vibrations and 1044 cm$^{(-1)}$ due to C=O vibrations.

IR spectrum of *Rhophalema Nomadica* Extract A also gave distinct active group footprints such as 3274 cm$^{(-1)}$ attributed to NH and OH vibrations, 1635 cm$^{(-1)}$ due to C=O vibrations of amide bonds, 1534 cm$^{(-1)}$ due to amide vibration and 1095 cm$^{(-1)}$ due to C---O vibrations. IR spectra of jellyfish extract/ PCL nanofibers exhibited peaks having strong correlation to the source materials.

IR spectrum results showed that jellyfish nanofibers exhibit peaks corresponding to NH and OH groups of the jellyfish extract part of the nanofibers and to C=O and O=C-C groups that belong to PCL molecules of the nanofibers. The IR spectrum supports a conclusion that the synthesized nanofibers comprise combinations of the two source materials.

Example 2C: Porosity measurements

Porosity was determined for various scaffolds comprising jellyfish extract/ PCL nanofibers, using ESEM images of scaffolds and ImageJ software. The empty areas between the nanofibers on the presumed outer layer of a nanofiber scaffold were measured. The sum of all empty areas was divided by the area of the image and multiplied by 100. The porosity results are discussed in the next section.

Example 2D: Contact angle

Contact angle for various scaffolds comprising jellyfish extract/ PCL nanofibers was determined using Rame-hart instrument co. Model 250 standard goniometer with DROPImage Advanced software v2.4. In order to measure nanofiber surface contact angle for hydrophobic or hydrophilic properties, a nanofiber scaffold sample was attached to a silicon wafer via carbon tape and placed on a setup stage. All the measurements were taken in a low humidity environment at room temperature. Porosity and contact angle were determined for samples made under various conditions using 35%/7% jellyfish extract/ PCL solution and are detailed in Table 4 below:

Table 4:

| Contact angle | Porosity (%) | kV | Flow (ml/hr) |
|---|---|---|---|
| 48 | 33 | 16 | 0.42 |
| 54 | 31.41 | 22.5 | 0.42 |
| 63.93 | 26.22 | 18.5 | 0.48 |
| 74 | 23.848 | 19 | 0.54 |
| 76.42 | 23.79 | 17 | 0.54 |

Contact angle measurements for jellyfish extract/ PCL nanofibers proved to be a difficult task due to several samples having an extremely hydrophilic surface. The rate of water drop absorbance was very high. All jellyfish extract/ PCL nanofiber scaffolds proved to be hydrophilic with some being extremely hydrophilic having low contact angle of below 30°. If a nanofiber sample is hydrophobic, for example pure PCL nanofiber, then the contact angle would be expected to be above 120°. Jellyfish extract/ PCL nanofibers in accordance with embodiments of the disclosure have contact angles far below 90° and are thus deemed to be hydrophilic.

After measuring porosity in each scaffold sample, a clear correlation between nanofiber porosity and surface contact angle became apparent. With increase in sample porosity there was decrease in surface contact angle. Nanofiber scaffolds with high degrees of porosity have higher affinity between the fibers and water molecules. All of the jellyfish-PCL nanofiber scaffolds exhibited surprisingly hydrophilic properties, considering that PCL-only nanofibers has hydrophobic characteristics with typical contact angles of between 120° and 140°.

Hydrophilic groups such as carboxylic acids, amide groups, amino acids side groups, hydroxyl groups and oligosaccharides are known to be present in the main components of the jellyfish extracts, collagen and Q-mucins. As such, the contact angle data is consistent with the outer layer of the jellyfish extract/ PCL nanofiber being enriched in jellyfish protein, as is supported by the results discussed below.

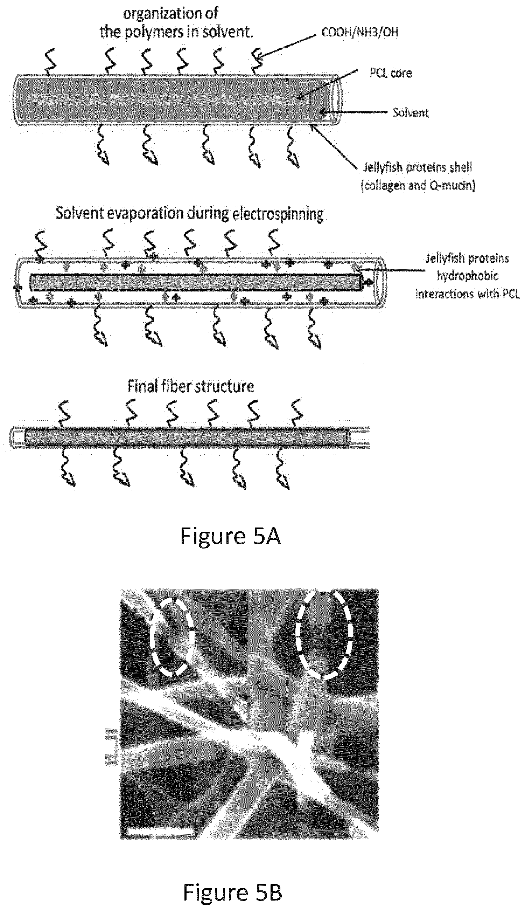

Figure 5A presents a suggested mechanism of jellyfish extract-PCL fiber formation during the electrospinning process, as well as a physical structure of the electrospun jellyfish extract-PCL nanofiber, which has an outer layer enriched in jellyfish extract and an inner core enriched in PCL.

In an embodiment of the disclosure, hydrophilic properties of the jellyfish extract-PCL nanofibers compared to the hydrophobic nature of the PCL co-polymer nanofibers can be explained by the formation of two phases during the fiber formation in the electrospinning process. The inner part of the fiber is mostly composed of the hydrophobic PCL and the outer shell is mostly composed of the jellyfish hydrophilic biomass (Figure 5A). The phases may be formed during jellyfish extract and PCL co-dispersion in the appropriate solvent, in this example acetic acid which serves as a mediator between jellyfish extract and PCL (Figure 5A). Acetic acid also induces positive charges on jellyfish extract, due to its acidic pH value that is lower than the pKa value of the jellyfish collagen and Q-mucin.

Figure 5B shows a JF-PCL image taken with Environmental Scanning Electron Microscopy (E-SEM) clearly showing an exposed inner core in some of the damaged jellyfish extract-PCL nanofibers. A dashed circle indicates the exposed inner core PCL material (scale bar 5 μm).

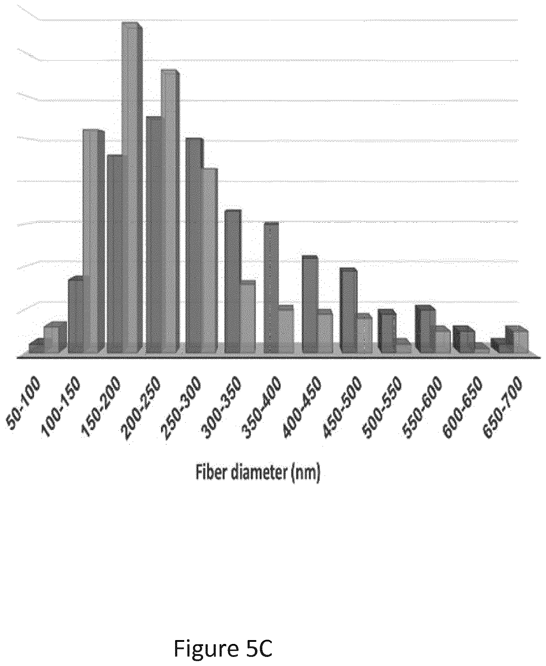

Further validation of the structure was done by means of selective enzymatic degradation of the biomass outer shell utilizing pepsin enzyme. Figure 5C shows jellyfish extract-PCL nanofiber thickness distribution before (dark gray) and after (light gray) enzymatic degradation by pepsin. As expected, the distribution of the nanofiber thickness shifted towards thinner fibers following pepsin degradation. The mean thickness of the pepsin-treated fibers decreased by approximately 70 nm, which corresponds to the outer portion of the nanofiber enriched in the jellyfish extract.

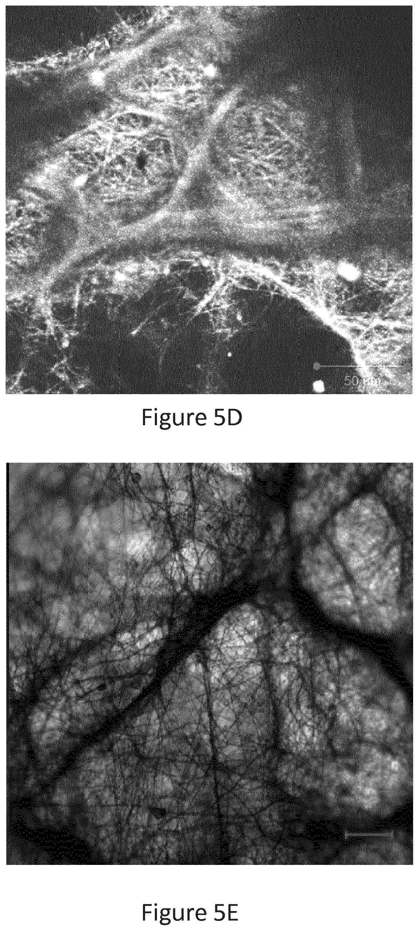

Figure 5D is a confocal fluorescence microscope image of DTAF labeled jellyfish extract-PCL nanofibers and Figure 5E is a confocal fluorescence microscope image of reference non-labeled fibers. Figure 5D shows a confocal fluorescence microscope image of jellyfish extract-PCL fibers labeled with, dichlorotriazinyl aminofluorescein (DTAF), a protein targets protein and glycoprotein via their amine, carboxy acids, and polysaccharides moieties. It is evident from the confocal fluorescence images of the DTAF labeled JF-PCL nanofibers that the outer surface of the fibers includes a protein/glycoprotein layer that is derived from the jellyfish extract. Control experiments (not shown) confirmed that there is no nonspecific labeling of PCL by DTAF or auto fluorescence of JF proteins.

Without being bound by theory, it is proposed that that Q-mucins in an acidic environment (of the acetic acid solution) expose a hydrophobic region, which can interact with PCL. During the electrospinning process, acetic acid rapidly evaporates, while the interaction between the acidified jellyfish extract and PCL polymer remains (Figure 5A).

Scaffold porosity percentage impacts surface wettability, as highly porous scaffolds have higher area/volume ratio allowing more contact sites between hydrophilic groups on nanofibers with a water drop. PCL nanofibers without jellyfish extract which also exhibit high porosity percentage have only hydrophobic interactions with a water drop, giving PCL scaffolds a higher (>120 degree) contact angle. Porosity of the different scaffold types was mainly controlled by applied voltage and adjusted flow rate of the polymer solution. Different voltage values required adjustment of the flow rate in order to produce stable Taylor cone and fine nanofibers.

Example 2E: Fluid handling properties

Fluid absorption was measured by cutting 2 cm x 2 cm square samples of jellyfish extract/ PCL nanofibers and weighing the samples to determine dry weight (W1). The samples were placed in saline solution (sodium chloride and calcium chloride standard medical solution) at 20°C and physically immersed using a glass weight. The jellyfish extract-PCL nanofiber samples were removed from saline solution and drained from freely draining fluid by means of absorbent paper then reweighed to obtain wet weight (W2). The weight of absorbed fluid was calculated as (W2-W1)/W1.

Dehydration rate of jellyfish extract-PCL nanofiber scaffolds was measured by placing dry weighed samples into deionized water for 30 min. Scaffolds then were removed from the liquid and dried for 1 min in air in order to remove the freely draining liquid and then reweighed. All the wet samples were put in an oven at 37°C. The loss of weight was monitored at equal time intervals until the samples reached their initial dry weight.

The liquid intake percentage relative to weight and dehydration rate for 5 scaffold samples comprising jellyfish extract- PCL nanofibers are shown in Table 5.

Table 5:

| Sample | Porosity | Liquid intake percentage | Dehydration rate g/min |
|---|---|---|---|
| A | 31.41%, | 448% | 0.0085 |
| B | 26.22%, | 417% | 0.006 |
| c | 23.79%, | 378% | 0.006 |
| D | 33% | 655% | 0.004 |
| E | 23.85% | 380% | 0.007 |

Absorption testing of different jellyfish extract-PCL nanofiber scaffolds showed that samples of 2 cm x 2 cm size have high liquid (saline solution) absorption capabilities. The highest absorption results were gained from sample D, with about 655% of liquid intake compared to its initial dry weight. Samples A, B, C and E showed similar results. Jellyfish extract-PCL nanofiber scaffolds possessed high fluid absorbing rate averaging 2-3 seconds until full scaffold saturation.

Dehydration rates of jellyfish extract-PCL nanofiber samples show that they posess rates 0.004-0.0085 g/min of liquid loss. Sample D showed the best results with the slowest rate of 0.004 g/min. Nevertheless the overall time for scaffold samples to reach their initial dry weight is about 15 min at 37°C .

Example 2F: Tensile strength measurements

Jellyfish extract- PCL nanofiber scaffolds from a variety of batches were collected. Collected nanofibers were cut into rectangular shape scaffolds with a typical height of 3 cm and a width of 0.5 cm. Scaffolds were put into a Lloyd tensile stress apparatus and measured against external applied force. The force vs. extension data were collected using a digital data acquisition hardware and software. Up to 5 specimens from each scaffold type were measured for statistical considerations. Tensile strength measurements were performed on 5 jellyfish extract/ PCL nanofiber scaffold types which differ from each other in nanofiber diameter and porosity percentage. Manufacturing process of jellyfish extract/ PCL nanofibers currently allows production of only randomly arranged nanofibers due to limitations of the electrospinning experimental setup described above. Results of Young's modulus measurements for each of the scaffolds having varied average fiber diameter are shown in Table 6 below

Table 6:

| Sample | Young's Modulus in Megapascals (MPa) | Average Fiber Diameter (nm) |
| --- | --- | --- |
| 1 | 7.38 | 338.7 |
| 2 | 7.57 | 258.3 |
| 3 | 7.85 | 197.1 |
| 4 | 7.074 | 422.1 |
| 5 | 8.63 | 186.63 |

As shown in the table, scaffolds comprising smallest diameter fibers showed higher Young's modulus values and possessed higher resistivity towards externally applied force.

Non-random nanofibers may be prepared by using the electrospinning system together with a spinning disk or cylinder collector. According to embodiments of the invention, nanofibers of lengths of longer than 1 cm and nanofibers of lengths longer than 1 meter may be formed.

Example 2G: Antimicrobial content

In order to test content of silver in nanofibers formed from jellyfish extract and PCL prepared as in Example 1D, jellyfish extract-PCL nanofibers were digested in a mixture of concentrated sulfuric and nitric acids to break down the jellyfish extract-PCL nanofiber and to dissolve all of the silver particles present on the nanofibers. The digest was filtered and diluted with deionized water and silver content was determined by atomic absorption spectrometry.

Fine silver nanoparticles were successfully produced on prepared jellyfish extract-PCL nanofiber scaffolds using silver ion concentrations 0.05M and 0.0005M. Silver nanoparticles were reduced to Ag° by the active groups present in Q-mucin (cysteine amino acids and oligosaccharides). The presence of silver particles on jellyfish nanofiber scaffolds was confirmed using ESEM, and Energy-dispersive X-ray spectroscopy (EDS, using HKL-EBSD and Oxford-EDS integrated analytical system) and the average silver particle size for various silver ion concentration solutions is listed in table 7 below:

Table 7:

| Silver Molarity | Average particle size (nm) | Standard Deviation (nm) |
| --- | --- | --- |
| 0.0005 | 85.695 | 24.8 |
| 0.005 | 70.87 | 27.3 |

Example 2H: Antimicrobial release

In order to test silver particle release of jellyfish extract-PCL nanofiber doped with silver particles, scaffolds comprising jellyfish extract-PCL nanofibers with silver particles were suspended in deionized water at a ratio of 1:100 (w/v), and placed into a temperature-controlled oven (37°C) for 12 hr. During this period aliquots were drawn on an hourly basis and the liquid was replaced to maintain a constant volume. Collected aliquots were analyzed by atomic absorption spectrometry.

Release of silver from jellyfish extract-PCL nanofibers essentially occurred almost to completion during the first hour of immersion in deionized water. Overall uptake of silver from solution into jellyfish extract-PCL scaffolds depends on the nanofiber characteristics such as active surface area of jellyfish extract-PCL scaffolds. Jellyfish extract-PCL scaffolds with a relatively large area and high nanofiber density could adsorb relatively more silver ions on their surfaces. Even after prolonged time (24 hours) in aqueous solution, jellyfish extract-PCL nanofiber retained some silver particles.

The retention indicates that the antimicrobial properties of silver-doped jellyfish scaffolds may be retained for a prolonged time.

In order to test release of a tetracycline from a scaffold comprising jellyfish extract-PCL, nanofiber doped with a tetracycline was placed in 5 ml deionized water for one hour. The deionized water was replaced with fresh deionized water on an hourly basis for an additional five hours. the concentration of tetracycline which was released from the scaffold into the solution was measured with a UV-visible light spectrometer where two aromatic peaks characteristic of tetracycline were clearly visible in the 260-450 nm range.

Release of tetracycline over time was measured and is represented in a graph in Fig. 3. There is a clear indication that nanofibers successfully absorbed more than 0.0001 M of tetracycline from a total solution concentration of 0.001M. The absorption expressed as mass of tetracycline/ mass of nanofibers was 0.44 mg tetracycline / 6.8 mg nanofiber. The initial release of tetracycline into aqua solvent from jellyfish extract-PCL nanofiber over a period of an hour is about 87%.

Release of tetracycline from the nanofibers was measured for 6 hr. After 4 hr the released tetracycline concentration fell to below 1% of its initial amount. The initial release of the tetracycline from the nanofiber scaffold was expected due to the hydrophilic and porous structure of the scaffold which induced the release of the mildly hydrophilic tetracycline into the buffer phosphate solution.

Examples 3A-3D: Testing of biological properties of jellyfish nanofibers

Example 3A: Cytotoxicity evaluation of jellyfish protein

*Rhophalema Nomadica* Extract A prepared according to Example 1B at concentrations of 100%, 30%, 10% and 3% (diluted with water and alginate) was added to wells containing normal human dermal fibroblast (NHDF) cells. The wells were incubated under 5% CO2, 37°±1° degrees, for 72 hr. Viability/proliferation was determined through fluorometric testing of cultured cells after addition of 10% alamar-blue dye into the culture media. The jellyfish extract at all concentrations was determined to be non-toxic.

Example 3B: Biodegradability of jellyfish extract

A biodegradability assay was performed in a MODA 6 Microbial Oxidative Degradation Analyzer, Saida, Japan. Biodegradability assay was conducted for a duration of 30 days, on jellyfish extract samples, each weighing 10 g. The jellyfish samples were prepared by mixing *Rhophalema Nomadica* Extract A with agarose biological cross-linker by mixing the following materials: 2% of agarose, 40% jellyfish extract, 20% glycerol and 38% water (by weight). Cellulose microcrystalline was used as reference material which has a high degree of biodegradability. Plastic composite material with low level of biodegradability was used as a negative control.

The biodegradation test was performed according to ISO 14855-2, determination of the ultimate aerobic biodegradability of plastic materials under controlled composting conditions by analysis of evolved carbon dioxide and by gravimetric measurement of carbon dioxide evolved in a laboratory-scale test. Samples were put in separate testing setup chambers which were heated to 58°C. Each sample containing chamber was attached to a reaction column which the samples degraded into. During the process the evolved ammonia and water were removed and the carbon dioxide was absorbed in soda lime (sodium hydroxide). Carbon dioxide evolution is measured by weight increase of the soda lime.

Final percentage of biodegradability level was calculated by weighing of the reaction column after each day, the gained weight of the column is interpolated as the sample weight loss. The testing indicates that jellyfish samples are highly biodegradable and meet the needed standard of over 90% degradability after 60 days.

Example 3C: Antimicrobial disk diffusion assay

Jellyfish extract-PCL nanofiber scaffolds doped with silver or tetracycline antimicrobial agents were prepared as described above and cut into squares of roughly 0.5 x 0.5 $cm^2$ in size. Petri dishes with agar + lysogeny broth (LB) bacterial growth medium were prepared by dissolving 3 g of agar and 2 g of LB in 150 ml of distilled water and pouring 30 ml of prepared solution into a Petri dish for further cool down and use. Bacterial medium was defrosted and grown by adding of 200 μl of gram positive bacteria type p479 pure medium to 9.8 ml of 10% LB solution and left overnight at 38-39°C in an incubator.

50 μl of bacterial medium was spread equally on a Petri dish and left in a fume hood for 10 min in order to dry the excessive liquid. The Petri dish was divided into 4 quarters with different materials acting as reference and test subjects. Quarter 1 contained reference with non-doped jellyfish extract-PCL nanofiber scaffold, Quarter 2 contained pure tetracycline, Quarters 3 and 4 contained tetracycline-doped jellyfish extract-PCL nanofiber scaffold. After the test and reference materials were put in the middle of each respective quarter, the Petri dish was put in the incubator for overnight at 40°C. Growth inhibition diameter around the tested sample was measured, representing the ability of the tested sample to release antimicrobial agent into the solid bacterial growth medium.

Various scaffold types were tested and the results of inhibition areas and diameter are tabulated in table 8 below:

Table 8:

| Sample name (same samples as shown in Table 5) | Scaffold surface area (cm$^2$) | Bacterial inhibition Diameter (cm) |
| --- | --- | --- |
| E | 0.4 | 1.5 |
| B | 0.25 | 1.3 |
| C | 0.56 | 1.6 |
| A | 0.25 | 1.5 |
| D | 0.16 | 1.6 |

Silver-loaded jellyfish extract-PCL nanofiber scaffolds were tested for inhibition as well. They were found to inhibit bacterial growth as well.

Example 3D: Cell proliferation evaluation

Jellyfish polymer-PCL nanofibers were assessed in a cell proliferation assay in order to assess biocompatibility of the jellyfish extract-PCL nanofibers with human epidermal tissue cells.

Jellyfish polymer-PCL nanofiber samples were produced as above, having a thickness of 50 micrometer ($\mu$m). Prepared nanofibers were cut into circular pieces of 1 cm in diameter and placed in specialized wells and sterilized under UV radiation. Subsequently the nanofiber samples were washed with the cell growth medium DMEM and left under a biological fume hood.

Fibroblast cells were grown and placed in incubator 37°C until further use. In order to separate fibroblast cells from the growth Petri dish, trypsin enzyme was added for 3 min and then washed with serum medium which disables trypsin. Subsequently fibroblast + serum solution was placed in a centrifuge under 300 G for 5 min in order to separate the cells as residue from the serum. Cells residue once again was added to the serum solution, now clean from trypsin, and the serum and cells solution was placed on an optical camera cell counter slide with 20 $\mu$l of triphenol blue dye which colors only the dead cells. After fibroblast cell number was counted, fibroblast cells and serum solution were taken and diluted until a volume in which 10 $\mu$l of serum solution contains approximately 50000 cells. 10 $\mu$l of cell and serum solution was placed on each jellyfish extract-PCL nanofiber sample and left for 15 min of adhesion time. After 15 min 0.5 ml of growth medium was added and jellyfish extract-PCL nanofiber samples were left in incubation for the duration of 7 days. Cell viability and proliferation were systematically checked after 3, 5 and 7 days of incubation.

The fibroblast cell proliferation assay showed overall non cytotoxicity of jellyfish extract-PCL nanofiber samples, indicating that jellyfish extract-PCL nanofiber may be used as a matrix to support cell growth.

The second type of cells chosen for a cell proliferation assay was cardiac cells. When cardiac cells are isolated from the heart muscle they gain a rounded morphology, lose their interactions with the surrounding cells, and their contractile proteins are lost or disorganized. Cardiac cells, as opposed to other types of cells such as fibroblasts, are highly sensitive to their surroundings and require a very supportive environment for their growth. A scaffold which supports cell growth and adherence should promote cardiac cells spreading, elongation, and reorganization of their contractile proteins.

Cardiac cells were isolated using left ventricles of 0-3 day old neonatal Sprague-Dawley rats. Hearts were harvested and cells were isolated using 6 cycles (30 min each) of enzyme digestion with collagenase type II (95 units (U)/mL; Worthington, Lakewood, NJ) and pancreatin (0.6 milligram (mg)/ml; Sigma-Aldrich) in Dulbecco's modified Eagle Medium (DMEM, ($CaCl_2 \cdot 2H_2O$ (1.8 mM), KCl (5.36 mM), $MgSO_4 \cdot 7H_2O$ (0.81 mM), NaCl (0.1 M), $NaHCO_3$ (0.44 mM), $NaH_2PO_4$ (0.9 mM)). After each round of digestion cardiac cells were centrifuged (600 G, 5 min) and re-suspended in culture medium composed of M-199 supplemented with 0.6 mM $CuSO_4 \cdot 5H2O$, 0.5 mM $ZnSO_4 \cdot 7H_2O$, 1.5 mM vitamin B12, 500 U/mL Penicillin and 100 mg/ml streptomycin, and 0.5% (v/v) FBS. To enrich the cardiomyocytes population, cardiac cells were suspended in culture medium with 5% FBS and pre-plated twice (45 min). Cardiac cell number viability and proliferation were determined by a hemocytometer and trypan blue exclusion assay.

$5X10^5$ cardiac cells were seeded onto the jellyfish extract-PCL nanofiber samples by adding 10 $\mu$l of the suspended cardiac cells followed by 1 hr incubation (37°C, 5% $CO_2$). Cell constructs were supplemented with culture medium (5% FBS) and further incubated.

Cardiac cell constructs were fixed and permeabilized in 100% cold methanol for 10 min, washed three times in Dulbecco's modified eagle medium (DMEM) based buffer and then blocked for 1 h at room temperature in DMEM-based buffer containing 2% FBS. The samples were then incubated with primary antibodies to detect $\alpha$-sarcomeric actinin (1:750, Sigma-Aldrich), washed three times, and incubated for 1 hr with Alexa Fluor 647 conjugated goat anti-mouse antibody (1:500; Jackson, West Grove, PA). For nuclei detection, the cardiac cells were incubated for 3 min with Hoechst 33258 (1:100; Sigma-Aldrich) and washed three times. Samples were visualized using a confocal microscope (Nikon Eclipse Ni).

To evaluate the bio-compatibility of the jellyfish extract-PCL nanofiber samples, cardiac cells were cultured within the scaffolds. The jellyfish extract-PCL nanofiber samples promoted cell spreading, elongation, encouraged cell-cell interactions and expression of massive actinin striation. These results indicate the positive potential of these scaffolds to accommodate cells and support their growth.

The cardiac cell proliferation assay showed overall non cytotoxicity of jellyfish extract-PCL nanofiber samples, and the possibility of interaction of cells with the jellyfish-extract-PCL, indicating that jellyfish extract-PCL nanofiber may be used as a matrix to support cell growth.

Example 4: Jellyfish nanofibers produced from a different jellyfish species

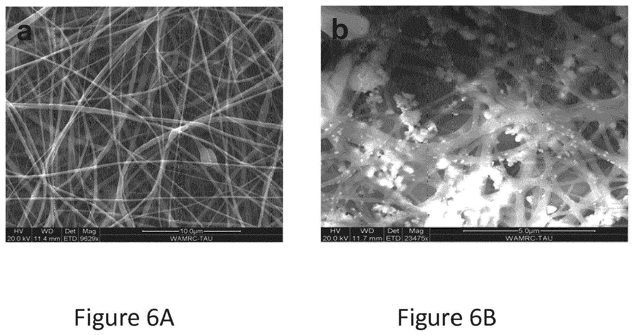

Nomura's jellyfish, *Nemopilema nomurai*, were collected near the south shore line of Korean sea and processed as described above for Extracts A. Figure 6A is an electron microscope micrograph of electrospun nanofibers made from Korean jellyfish extracts, and Figure 6B is an electron microscope micrograph of silver nanoparticles' nucleation on nanofibers made from Korean jellyfish extracts. The produced jellyfish extract material was used in the electrospinning process for wound dressing production. The nanofibers appeared to have the same properties as nanofibers made from material of Rhophalema Nomadica extract A and photomicrographs of the Electrospun nanofibers and silver nanoparticles' nucleation on the nanofibers were also similar to those obtained with material of Rhophalema Nomadica extract A.

Example 5: Testing of jellyfish nanofiber wound dressing

Example 5A: A wound dressing material made with jellyfish nanofibers tested on a murine wound healing model

A pilot animal test was performed using 5 mice according to an internally approved protocol (Tel Aviv University Protocol 04-16-046). A test group of 5 mice underwent a surgical procedure in which two circular and full thickness wounds were inflicted on each mouse. One wound acted as a reference wound wherein a simple wound dressing (gauze) was applied, whereas on the second wound we applied jelly fish extract-PCL nanofiber antimicrobial wound dressing laced with silver nanoparticles. The wounds were systematically measured and re bandaged until complete re epithelization. The healing rate of each wound for each mouse was optically measured to evaluate wound closure.

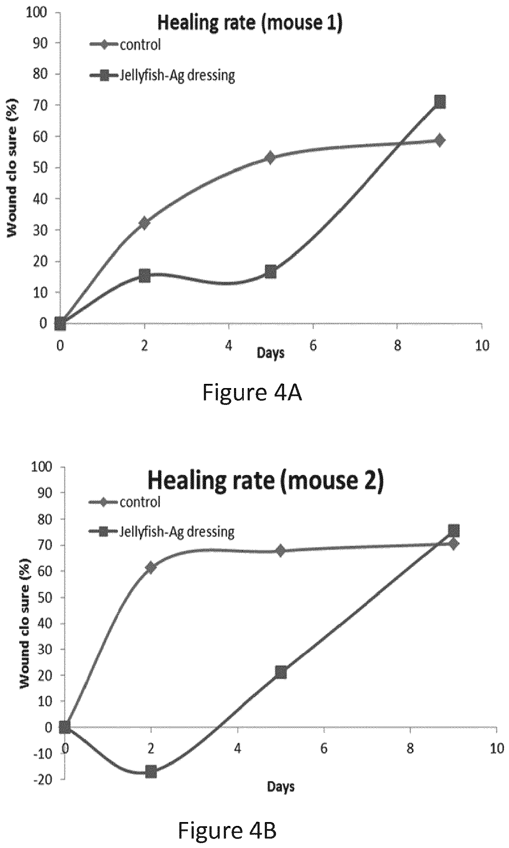

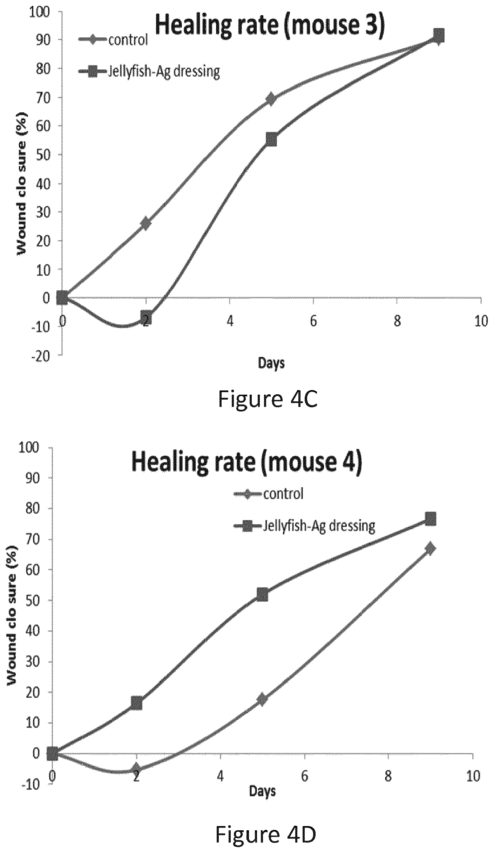

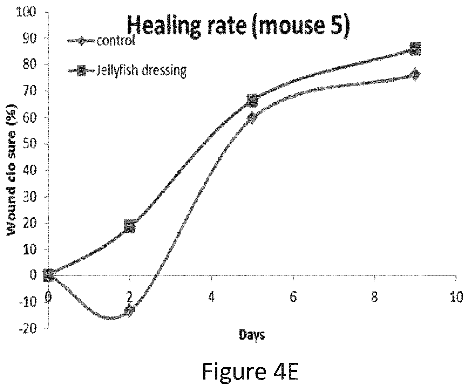

Figures 4A-4E each depict for a different one of the five mice the evaluated wound closure (%) over time (days).

Some wounds initially healed better under the simple wound dressing, as is shown in Figures 4A, 4B and 4C. However, by day 9 all five mice demonstrated better healing of the wounds when the wounds were bandaged with jelly fish extract-PCL nanofiber antimicrobial wound dressing laced with silver nanoparticles.

The wounds were photographed and the images were processed by the Image J program. Each measurement is an average area taken from several photographs of the same wound. The rate of wound healing was calculated by Equation (1):

$$(A_{t=0} - A_{t=x}) * 100 / A_{t=0} = \text{wound healing rate} \quad (1)$$

In all mice with tested jellyfish-Ag dressing no skin extraction was observed (except mouse 3 wounds). Jellyfish nanofiber scaffolds acted as fixating patches that prevented unnecessary movement of the skin.

Wound healing evaluation: Table 9 summarizes the results of testing wound healing in respect of histological measurements of the wounds.

Table 9:

| Cell Type | M1 JF | M1 ctrl | M2 JF | M2 ctrl | M3 JF | M3 ctrl | M4 JF | M4 ctrl | M5 JF | M5 ctrl |
|---|---|---|---|---|---|---|---|---|---|---|
| Inflammatory cells | + | ++++ | + | ++++ | +++ | +++ | +++ | +++ | + | +++ |

14

(continued)

| Cell Type | M1 JF | M1 ctrl | M2 JF | M2 ctrl | M3 JF | M3 ctrl | M4 JF | M4 ctrl | M5 JF | M5 ctrl |
|---|---|---|---|---|---|---|---|---|---|---|
| Collagen fibers | +++ | +++ | +++ | + | ++ | ++ | ++++ | ++++ | +++ | +++ |

| Legend: |
|---|
| M1 JF: Jellyfish extract dressing embedded with silver nanoparticles, on Mouse 1 |
| M1 Ctrl: Control dressing without Jellyfish extract on Mouse 1 |
| +: mild; ++: moderate; +++: high; ++++: very high |

Control wounds in tended to show a higher presence of inflammatory cells, implying that the wound healing process is incomplete. In addition, the wound in the controls may have been infected with bacteria that caused inflammation which in turn may stall the healing process. By contrast, wounds with the tested jellyfish extract and silver dressing exhibited minimal presence of inflammatory cells, except in mice 3 and 4, where wound size was large and the wound healing process was not completed within the timeframe of the experiment.

In addition, in mouse 1 and mouse 5, the wounds with jellyfish extract and silver dressing exhibited high number of collagen formations with structured texture in the upper epidermal layer and dermis layer, whereas in the control wounds of the same mice collagen formations are absent from the upper dermis regions, implying that the late healing process had not commenced in the control wounds.

The would in mouse 2 treated with jellyfish extract and silver dressing exhibited a high number of collagen formations with structured texture in the lower epidermal layer and upper dermis layer, whereas in the control wound, collagen formations are absent from the lower epidermal layer but are present in the upper dermis regions.

Mouse 3 and 4 showed in wound with jellyfish extract and silver dressing and control wound similar collagen fiber formations.

Conclusions:

The silver and jellyfish extract dressing didn't cause any apparent toxic effect during wound healing process.

Epidermal and dermal layers were fully formed similarly to the natural healing process.

The wounds covered with a silver and jellyfish extract dressing also showed presence of a more complete stratum corneum cell layer.

Tested wounds covered with a silver and jellyfish extract dressing exhibited less inflammatory cells presence, indicating that the healing process is generally more complete than in wounds covered with a regular dressing.

Treatment of wounds with the silver and jellyfish extract dressing exhibits enhanced efficacy in the healing of the wounds.

Example 5B: A composite jellyfish wound dressing material tested on a porcine wound healing model

An animal experiment on a porcine wound healing model was conducted to evaluate the effect of a 3-layer and 4-layer composite jellyfish nanofiber wound dressing on healing progress of a wound and to compare it to commercial smart wound dressing.

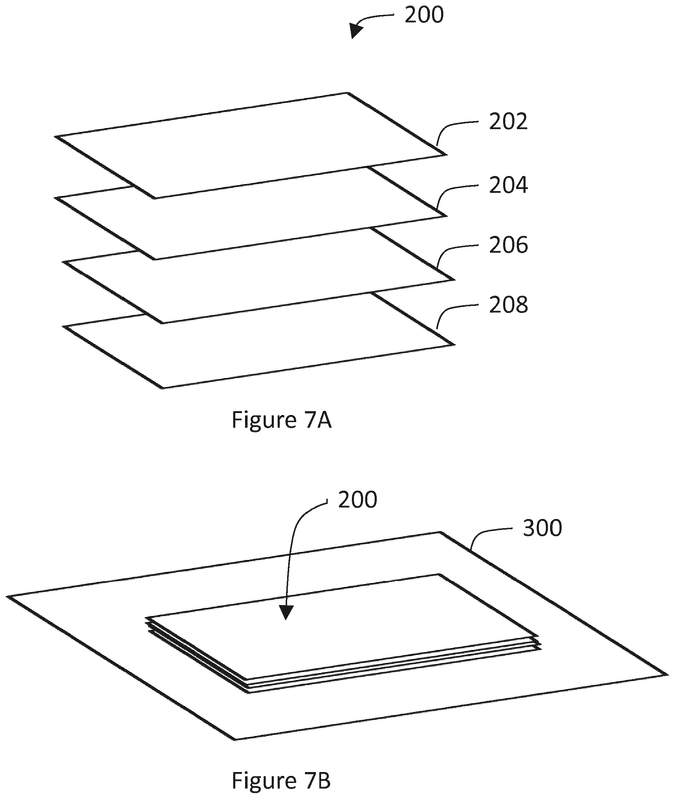

Fig. 7 schematically shows an exploded view of a four-layer composite wound dressing 200 in accordance with an embodiment of the disclosure, comprising: a contact layer 202 comprising a jellyfish nanofiber scaffolding mat; an antimicrobial layer 204 comprising a jellyfish nanofiber scaffolding mat loaded with an antimicrobial agent, by way of example silver nanoparticles; a hydrogel layer 206 comprising a jellyfish nanofiber scaffolding mat loaded with a hydrogel, by way of example sodium alginate hydrogel; and an external cover layer 208 comprising a jellyfish nanofiber scaffolding mat.

Optionally, each layer is produced separately then arranged together in a sandwich-like manner to produce a single compound wound dressing. Optionally, external cover layer 208 and/or contact layer 202 is electrospun directly onto antimicrobial layer 204 and/or hydrogel layer 206. A three-layer composite dressing (not shown) in accordance with an embodiment of the disclosure comprises antimicrobial layer 204 or hydrogel layer 206 sandwiched between contact layer 202 and external cover layer 208.

Fig. 7B schematically shows a completed four-layer composite dressing 200 placed over a wound on a skin 300 of a subject.

Three animals were acclimatized for a week before the start of experiment. The animals were fasted overnight and giver 0.05 mg/kg bufernuprin. On the operation day the animals were given ksezilen 2 mg/kg intramuscularly 15 min before sedating with ketamine 10 mg/kg IM, followed by masking down with 1-3% isofluorane and intubated with a size 7.0 endotracheal tube. Aseptic techniques were strictly practiced throughout the procedure. The hair on the back of the pigs was shaved and vacuumed. The skin was cleaned with 1% cetrimide wash, 0.05% chlorhexidine, and finally with 1% povidone- iodine. 16 rectangular 1.5x1.5 cm full thickness wounds were created on the dorsal aspect on each pig's skin by scalpel cutting. The wound thickness was approximately 8-9 mm. Each wound was treated with appropriate wound dressing with area of 4 cm$^2$.

On each pig the wounds were divided to 4 time groups; Day3, Day6, Day9 and Day 12. Each time group was divided into 4 wounds with different applied wound dressing. Each time group contained the next wound dressings:

Composite dressing A, a four-layer composite wound dressing in accordance with an embodiment of the disclosure comprising a contact layer comprising a jellyfish nanofiber scaffolding mat, a silver layer comprising a jellyfish nanofiber scaffolding mat loaded with silver nanoparticles, a hydrogel layer comprising a jellyfish nanofiber scaffolding mat loaded with a sodium alginate hydrogel, and an external cover layer comprising a jellyfish nanofiber scaffolding mat;

Composite dressing B comprising: a contact layer, a silver layer and an external cover layer;
Composite dressing C comprising: a contact layer, a hydrogel layer and an external cover layer;
Composite dressing D: A Silvercell commercial dressing by Systagenix™ comprising cellulose microfibers with calcium alginate and silver ion coating.

The wound were observed systematically on the day 3, 6, 9 and 12. In each day the wound were photographed, measured and new appropriate wound dressings were applied on the previously designated wound. On each experimental day, four wound samples from the appropriate time group were collected for histopathology analysis. For example on Day 3 all the wounds of time group day 3 were collected from all the animals. The average would closure for each dressing at each observed day is shown in the following table 10:

Table 10

| Average wound closure % | | | | |
|---|---|---|---|---|
| Day | Dressing A | Dressing B | Dressing C | Dressing D |
| 12 | 85.89484 | 69.64226 | 69.58652 | 85.77973 |
| 9 | 55.28646 | 46.73348 | 48.16017 | 49.76946 |
| 6 | 34.80615 | 34.89308 | 36.79234 | 37.34997 |
| 3 | 13.46418 | 22.47153 | 12.34174 | 10.65538 |

The 4 layer compound jellyfish nanofiber wound dressing (Dressing A) showed that it's healing capabilities are similar to commercial Silvercell dressing (Dressing D). The 3 layer jellyfish nanofiber smart wound dressings (Dressings B and C) also demonstrated satisfactory wound healing results with wound closure % of -70% after 12 days of experiment.

The examples above describe elecrospun fibers made from PCL (as a representative biodegradable polymer not derived from jellyfish) and jellyfish extract. Similarly, the electrospun fibers may be made from PCL or another biodegradable polymer not derived from jellyfish.

In an embodiment of the disclosure, the jellyfish extract comprises an alcohol extract of jellyfish biomass. Optionally, the alcohol is ethanol. Optionally, the jellyfish extract is extracted by incubating jellyfish biomass in ethanol at a concentration of between 70% and 100% ethanol.

In an embodiment of the disclosure, the jellyfish biomass comprises a bell portion of a jellyfish.

In an embodiment of the disclosure, the nanofiber comprises an inner core and an outer layer, wherein the inner core is enriched in the electrospinnable polymer relative to the outer layer, and the outer layer is enriched in the jellyfish extract relative to the inner core.

In an embodiment of the disclosure, the nanofiber has a diameter of between 150 and 900 nanometers.

The nanofiber according to claim 1, wherein the electrospinnable polymer comprises a selection of one or more of: PCL (polycaprolactone), DegraPol® (degradable block polyesterurethane), Pellethane 2363-80A (degradable polyetherurethane), Poly(3 - hydroxybutyrate -co-3-hydroxyvalerate), Poly(3- hydroxybutyrate), Poly(D,L-lactide -co - trimethylene carbonate, Poly(D,L-lactide)-poly(ethylene glycol), Mw = 51 kDa, Poly(L-lactic acid)-co-(caprolactone), 70:30 Mw 150 kDa, Poly(L-lactic acid)-co-(glycolic acid), 70:30, Poly(propylene carbonate), Mw 699 000 g/mol, Poly(?-stearyl-L-glutamate), Poly-L-lactide 1.09 dL and Poly[(alanino ethylester)0.67 (glycino ethyl ester)0.33 phosphazene. Optionally, the electrospinnable polymer comprises PCL (polycaprolactone).

In an embodiment of the disclosure, the nanofiber comprises: a jellyfish extract comprising a Q-mucin; a non-jellyfish electrospinnable polymer; and an antimicrobial agent,the nanofiber having a diameter of between 150 and 900 nanometers.

There is provided a nanofiber scaffold mat comprising nanofibers as described above.

In an embodiment of the disclosure, the nanofiber scaffold mat further comprises an antimicrobial agent. Optionally, the antimicrobial agent comprises silver nanoparticles. Optionally, the antimicrobial agent comprises a tetracycline.

In an embodiment of the disclosure, the nanofiber scaffold mat comprises a hydrogel.

In an embodiment of the disclosure, the nanofiber scaffold mat as described above is configured to be used as wound dressing material, tissue engineering scaffold matrix, cosmetics, filtration membranes, catalysis, medical textile, drug delivery and protective textile.

There is also provided a composite wound dressing comprising: a contact layer comprising a nanofiber scaffold mat; a nanofiber scaffold mat comprising an antimicrobial agent and/or a nanofiber scaffold mat according comprising a hydrogel; and external layer comprising a nanofiber scaffold mat.

There is also provided a method for manufacture of a nanofiber as defined in claim 1, the method comprising: providing a solution comprising a jellyfish extract; providing a solution comprising an non-jellyfish electrospinnable polymer; mixing the jellyfish extract solution with the electrospinnable polymer solution to form a mixture; and elecrospinning the mixture in an electrospinning system.

In an embodiment of the disclosure, the electrospinning is conducted under the following conditions: applying a voltage of between 10 kV and 22.5 kV between a needle and a collector; a distance between the needle end and the collector is between 10 cm and 20cm; and a flow of the mixture from the needle is between 2 microliters/minute and 10 microliters/minute.

In an embodiment of the disclosure, the ratio by dry weight of jellyfish extract to electrospinnable polymer in the mixture is between 5:1 and 10:1.

In an embodiment of the disclosure, the jellyfish extract is an alcohol extract of jellyfish biomass. Optionally, the alcohol is ethanol. Optionally, the jellyfish extract is extracted by incubating jellyfish biomass in ethanol at a concentration of between 70% and 100% ethanol.

In an embodiment of the disclosure, the jellyfish biomass comprises a bell portion of a jellyfish.

In an embodiment of the disclosure, the electrospinnable polymer comprises a selection of one or more of: PCL (polycaprolactone), DegraPol® (degradable block polyesterurethane), Pellethane 2363-80A (degradable polyetherurethane), Poly(3 - hydroxybutyrate -co-3-hydroxyvalerate), Poly(3- hydroxybutyrate), Poly(D,L-lactide -co - trimethylene carbonate, Poly(D,L-lactide)-poly(ethylene glycol), Mw = 51 kDa, Poly(L-lactic acid)-co-(caprolactone), 70:30 Mw 150 kDa, Poly(L-lactic acid)-co-(glycolic acid), 70:30, Poly(propylene carbonate), Mw 699 000 g/mol, Poly(?-stearyl-L-glutamate), Poly-L-lactide 1.09 dL and Poly[(alanino ethylester)0.67 (glycino ethyl ester)0.33 phosphazene. Optionally, the electrospinnable polymer comprises PCL (polycaprolactone).

**Claims**

1. A nanofiber comprising a jellyfish extract and at least one non-jellyfish-derived electrospinnable polymer, wherein the jellyfish extract comprises a jellyfish-derived collagen and a jellyfish-derived Q-mucin, **characterized in that** the nanofiber is produced by electro spinning.

2. The nanofiber according to claim 1, wherein the jellyfish extract comprises an alcohol extract of jellyfish biomass extracted by incubating jellyfish biomass in an alcohol.

3. The nanofiber according to claim 1, wherein between 1% and 15% of a dry weight of the jellyfish extract consists of the Q-mucin.

4. The nanofiber according to claim 1, wherein between 1% and 3% of a dry weight of the jellyfish extract consists of the Q-mucin.

5. The nanofiber according to claim 1 comprising an inner core and an outer layer, wherein the inner core is enriched in the electrospinnable polymer relative to the outer layer, and the outer layer is enriched in the jellyfish extract relative to the inner core.

6. The nanofiber according to claim 1, wherein the nanofiber has a diameter of between 150 and 900 nanometers.

7. The nanofiber according to claim 1, wherein the electrospinnable polymer is selected from the group consisting of:

PCL (polycaprolactone), degradable block polyesterurethane, degradable polyetherurethane, Poly(3 - hydroxybutyrate -co-3-hydroxyvalerate), Poly(3- hydroxybutyrate), Poly(D,L-lactide -co - trimethylene carbonate, Poly(D,L-lactide)-poly(ethylene glycol), Mw = 51 kDa, Poly(L-lactic acid)-co-(caprolactone), 70:30 Mw 150 kDa, Poly(L-lactic acid)-co-(glycolic acid), 70:30, Poly(propylene carbonate), Mw 699 000 g/mol, Poly(y-stearyl-L-glutamate), Poly-L-lactide 1.09 dL and Poly[(alanino ethylester)0.67 (glycino ethyl ester)0.33 phosphazene.

8. The nanofiber according to claim 7, wherein the electrospinnable polymer comprises PCL (polycaprolactone).

9. The nanofiber according to any one of the preceding claims further comprising an antimicrobial agent.

10. The nanofiber according to claim 9, wherein the antimicrobial agent comprises silver nanoparticles.

11. The nanofiber according to claim 9, wherein the antimicrobial agent comprises a tetracycline.

12. A nanofiber scaffold mat comprising a nanofiber according to any one of claims 1-11.

13. A nanofiber scaffold mat according to claim 12 further comprising a hydrogel.

14. A composite wound dressing (200) comprising:

a contact layer (202) comprising a nanofiber scaffold mat comprising a nanofiber according to claim 1;
an intermediate layer (204, 206) comprising a nanofiber scaffold mat comprising a nanofiber according to any one of claims 9-11 or a nanofiber scaffold mat according to claim 13; and
an external layer (208) comprising a nanofiber scaffold mat comprising a nanofiber according to claim 1.

15. A composite wound dressing (200) comprising:

a contact layer (202) comprising a nanofiber scaffold mat comprising a nanofiber according to claim 1;
an antimicrobial layer (204) comprising a nanofiber scaffold mat comprising a nanofiber according to any one of claims 9-11;
a hydrogel layer (206) comprising a nanofiber scaffold mat according to claim 13; and
an external layer (208) comprising a nanofiber scaffold mat comprising a nanofiber according to claim 1.

**Patentansprüche**

1. Nanofaser, umfassend einen Quallenextrakt und mindestens ein nicht von einer Qualle abgeleitetes elektrospinnbares Polymer, wobei der Quallenextrakt ein von einer Qualle abgeleitetes Kollagen und ein von einer Qualle abgeleitetes Q-Mucin umfasst, **dadurch gekennzeichnet, dass** die Nanofaser durch Elektrospinnen hergestellt ist.

2. Nanofaser nach Anspruch 1, wobei der Quallenextrakt einen Alkoholextrakt von Quallen-Biomasse umfasst, der durch Inkubation von Quallen-Biomasse in einem Alkohol extrahiert wird.

3. Nanofaser nach Anspruch 1, wobei zwischen 1% und 15% des Trockengewichts des Quallenextrakts aus dem Q-Mucin bestehen.

4. Nanofaser nach Anspruch 1, wobei zwischen 1% und 3% des Trockengewichts des Quallenextrakts aus dem Q-Mucin bestehen.

5. Nanofaser nach Anspruch 1, umfassend einen inneren Kern und eine äußere Schicht, wobei der innere Kern relativ zu der äußeren Schicht mit dem elektrospinnbaren Polymer angereichert ist und die äußere Schicht relativ zu dem inneren Kern mit dem Quallenextrakt angereichert ist.

6. Nanofaser nach Anspruch 1, wobei die Nanofaser einen Durchmesser zwischen 150 und 900 Nanometer aufweist.

7. Nanofaser nach Anspruch 1, wobei das elektrospinnbare Polymer ausgewählt ist aus der Gruppe bestehend aus: PCL (Polycaprolacton), abbaubares Blockpolyesterurethan, abbaubares Polyetherurethan, Poly(3-Hydroxybutyrat-co-3-hydroxyvalerat), Poly(3-Hydroxybutyrat), Poly(D,L-Lactid-co-trimethylencarbonat, Poly(D,L-Lactid)-poly(ethylenglykol), Mw = 51 kDa, Poly(L-Milchsäure)-co-(Caprolacton), 70:30 Mw 150 kDa, Poly(L-Milchsäure)-co-(glykolsäure), 70:30, Polypropylencarbonat), Mw 699 000 g/mol, Poly(y-Stearyl-L-glutamat), Poly-L-L-Lactid 1,09 dL und Poly[(Alanin-ethylester)0,67(Glycin-ethylester)0,33 Phosphazen.

8. Nanofaser nach Anspruch 7, wobei das elektrospinnbare Polymer PCL (Polycaprolacton) umfasst.

9. Nanofaser nach einem der vorstehenden Ansprüche, weiter umfassend ein antimikrobielles Mittel.

10. Nanofaser nach Anspruch 9, wobei das antimikrobielle Mittel Silbernanopartikel umfasst.

11. Nanofaser nach Anspruch 9, wobei das antimikrobielle Mittel ein Tetracyclin umfasst.

12. Nanofasergerüstmatte, umfassend eine Nanofaser nach einem der Ansprüche 1-11.

13. Nanofasergerüstmatte nach Anspruch 12, weiter umfassend ein Hydrogel.

14. Verbundstoff-Wundverband (200), umfassend:

   eine Kontaktschicht (202) umfassend eine Nanofasergerüstmatte, die eine Nanofaser nach Anspruch 1 umfasst;
   eine Zwischenschicht (204, 206) umfassend eine Nanofasergerüstmatte, die eine Nanofaser nach einem der Ansprüche 9-11 oder eine Nanofasergerüstmatte nach Anspruch 13 umfasst; und
   eine äußere Schicht (208) umfassend eine Nanofasergerüstmatte, die eine Nanofaser nach Anspruch 1 umfasst.

15. Verbundstoff-Wundverband (200), umfassend:

   eine Kontaktschicht (202) umfassend eine Nanofasergerüstmatte, die eine Nanofaser nach Anspruch 1 umfasst;
   eine antimikrobielle Schicht (204) umfassend eine Nanofasergerüstmatte, die eine Nanofaser nach einem der Ansprüche 9-11 umfasst;
   eine Hydrogelschicht (206) umfassend eine Nanofasergerüstmatte nach Anspruch 13; und
   eine äußere Schicht (208) umfassend eine Nanofasergerüstmatte, die eine Nanofaser nach Anspruch 1 umfasst.

## Revendications

1. Nanofibre comprenant un extrait de méduse et au moins un polymère électrofilable non dérivé de méduse, dans laquelle l'extrait de méduse comprend un collagène dérivé de méduse et une Q-mucine dérivée de méduse, **caractérisée en ce que** la nanofibre est produite par électrofilage.

2. Nanofibre selon la revendication 1, dans laquelle l'extrait de méduse comprend un extrait alcoolique de biomasse de méduse extrait par incubation de la biomasse de méduse dans un alcool.

3. Nanofibre selon la revendication 1, dans laquelle entre 1% et 15 % d'un poids sec de l'extrait de méduse consiste en la Q-mucine.

4. Nanofibre selon la revendication 1, dans laquelle entre 1% et 3% d'un poids sec de l'extrait de méduse consiste en la Q-mucine.

5. Nanofibre selon la revendication 1 comprenant un noyau interne et une couche extérieure, dans laquelle le noyau interne est enrichi du polymère électrofilable par rapport à la couche extérieure, et la couche extérieure est enrichie de l'extrait de méduse par rapport au noyau interne.

6. Nanofibre selon la revendication 1, dans laquelle la nanofibre a un diamètre compris entre 150 et 900 nanomètres.

7. Nanofibre selon la revendication 1, dans laquelle le polymère électrofilable est sélectionné à partir du groupe constitué par : PCL (polycaprolactone), polyesteruréthane bloc dégradable, polyétheruréthane dégradable, Poly(3-hydroxybutyrate-co-3-hydroxyvalérate), Poly(3-hydroxybutyrate), Poly(D,L-lactide-co-triméthylène carbonate), Poly(D,L-lactide)-poly(éthylène glycol), Mw = 51 kDa, Poly(L-acide lactique)-co-(caprolactone), 70:30 Mw 150 kDa, Poly(L-acide lactique)-co-(acide glycolique), 70:30, Poly(carbonate de propylène), Mw 699 000 g/mol, Poly(y-stéaryl-L-glutamate), Poly-L-lactide 1,09 dL et Poly[(alaninoéthylester)0,67 (glycinoéthylester)0,33 phosphazène.

8.  Nanofibre selon la revendication 7, dans laquelle le polymère électrofilable comprend du PCL (polycaprolactone).

9.  Nanofibre selon l'une quelconque des revendications précédentes, comprenant en outre un agent antimicrobien.

10. Nanofibre selon la revendication 9, dans laquelle l'agent antimicrobien comprend des nanoparticules d'argent.

11. Nanofibre selon la revendication 9, dans laquelle l'agent antimicrobien comprend une tétracycline.

12. Matrice d'échafaudage de nanofibre comprenant une nanofibre selon l'une quelconque des revendications 1-11.

13. Matrice d'échafaudage de nanofibre selon la revendication 12, comprenant en outre un hydrogel.

14. Pansement composite (200) comprenant :

une couche de contact (202) comprenant une matrice d'échafaudage de nanofibre comprenant une nanofibre selon la revendication 1 ;
une couche intermédiaire (204, 206) comprenant une matrice d'échafaudage de nanofibre comprenant une nanofibre selon l'une quelconque des revendications 9-11 ou une matrice d'échafaudage de nanofibre selon la revendication 13 ; et
une couche externe (208) comprenant une matrice d'échafaudage de nanofibre comprenant une nanofibre selon la revendication 1.

15. Pansement composite (200) comprenant :

une couche de contact (202) comprenant une matrice d'échafaudage de nanofibre comprenant une nanofibre selon la revendication 1 ;
une couche antimicrobienne (204) comprenant une matrice d'échafaudage de nanofibre comprenant une nanofibre selon l'une quelconque des revendications 9-11 ;
une couche d'hydrogel (206) comprenant une matrice d'échafaudage de nanofibre selon la revendication 13 ; et
une couche externe (208) comprenant une matrice d'échafaudage de nanofibre comprenant une nanofibre selon la revendication 1.

Figure 1A

Figure 1B

Figure 2A

Figure 2B

**14 cm**

Figure 2C

**16 cm**

Figure 2D

Figure 3

Figure 4A

Figure 4B

Figure 4C

Figure 4D

Figure 4E

organization of
the polymers in solvent.

COOH/NH3/OH

PCL core

Solvent

Jellyfish proteins shell
(collagen and Q-mucin)

Solvent evaporation during electrospinning

Jellyfish proteins
hydrophobic
interactions with PCL

Final fiber structure

## Figure 5A

## Figure 5B

Fiber diameter (nm)

Figure 5C

Figure 5D

Figure 5E

Figure 6A

Figure 6B

200

202

204

206

208

Figure 7A

200

300

Figure 7B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62320547

**Non-patent literature cited in the description**

- **JANAIRO R.R.R et al.** Mucin covalently bonded to microfibers improves the patency of vascular grafts. *TISSUE ENGINEERING,* 01 January 2014, vol. 20, 285-293